# EUROPÄISCHE PATENTSCHRIFT

(11) **EP 1 519 906 B1**
(45) Veröffentlichungstag und Bekanntmachung des Hinweises auf die Patenterteilung: **25.02.2009**
(21) Anmeldenummer: 03763642.0
(22) Anmeldetag: 14.06.2003
(51) Int. Cl.: C07C 41/42

(54) **VERFAHREN ZUR HERSTELLUNG VON HOCHREINEM METHYL-TERT.-BUTYLETHER**
METHOD FOR PRODUCING HIGH-PURITY METHYL TERT-BUTYL ETHER
PROCEDE POUR LA PRODUCTION DE METHYL-TERT-BUTYLETHER DE GRANDE PURETE

(30) Priorität: 10.07.2002 DE 10231051
(43) Veröffentlichungstag der Anmeldung: 06.04.2005
(73) Patentinhaber: Evonik Oxeno GmbH, 45772 Marl (DE)
(72) Erfinder: OBENAUS, Fritz, 45770 Marl (DE); DROSTE, Wilhelm, 45770 Marl (DE); SCHOLZ, Bernhard, 45768 Marl (DE); NIERLICH, Franz, 45768 Marl (DE); MALZKORN, Rainer, Mobile, Alabama 36695 (US); PETERS, Udo, 45770 Marl (DE); PRAEFKE, Jochen, 45770 Marl (DE); FILIPIAK, Richard, 45772 Marl (DE); NEUMEISTER, Joachim, 45721 Haltern am See (DE)
(74) Vertreter: Hirsch, Hans-Ludwig
(86) Internationale Anmeldenummer: PCT/EP2003/006300
(87) Internationale Veröffentlichungsnummer: WO 2004/007412

(56) Entgegenhaltungen:
- EP-A- 0 466 954
- US-A- 5 210 327
- US-A- 5 609 734

## Beschreibung

Die Erfindung betrifft ein Verfahren zur Herstellung von Methyl-tert.-butylether (MTBE) in Qualitäten, die für organische Synthesen und zur Verwendung als Speziallösemittel geeignet sind, aus MTBE in Kraftstoffqualität.

Technisches MTBE ist eine gefragte Komponente in Kraftstoffen für Ottomotoren zur Erhöhung der Octanzahl. Dabei werden hinsichtlich der Reinheit keine allzu hohen Anforderungen gestellt. Der Gehalt an Methanol und tert.-Butanol darf jeweils bis zu 1 Massen-% betragen. Weiterhin kann technisches MTBE bis zu 0,5 Massen-% C₄- bis C₈-Kohlenwasserstoffe und bis zu 500 ppm Wasser enthalten (Produktinformation MTBE, Oxeno GmbH, 3/2001).

Für den Pharmabereich und in der Analytik werden als Löse- und Extraktionsmittel hochreine Solvenzien verwendet. Bei der Herstellung von metallorganischen Verbindungen, beispielsweise Grignard-Verbindungen, und deren Umsetzungen werden nichtprotische Lösungsmittel mit Donoreigenschaften eingesetzt. Dazu werden häufig niedrige Ether, wie beispielsweise Diethylether, Diisopropylether, Tetrahydrofuran oder MTBE verwendet. Die drei zuerst genannten Ether haben den Nachteil einer niedrigen Zündtemperatur und eines weiten Explosionsbereichs. Weiterhin bilden sie in Gegenwart von Sauerstoff äußerst leicht Peroxide. Beispiele von Unfällen mit Peroxiden, sogar mit Todesfolge, sind aus der einschlägigen Literatur bekannt. Bei den meisten Anwendungen kann anstatt der zuerst genannten Ether MTBE eingesetzt werden. MTBE hat den Vorteil, dass es keine Peroxide bildet.

MTBE wird aus isobutenhaltigen C₄-Olefingemischen, beispielsweise dem C₄-Schnitt aus Steamcrackern oder FCC-Einheiten, gewonnen. Diese Gemische bestehen im Wesentlichen aus Butadien, Isobuten, 1-Buten und den beiden 2-Butenen, sowie den gesättigten Kohlenwasserstoffen Isobutan und n-Butan. Übliche weltweit ausgeübte Aufarbeitungsverfahren für solche C₄-Schnitte umfassen folgende Schritte: Zuerst wird der größte Teil des Butadiens entfernt. Kann Butadien gut vermarktet werden oder besteht ein Eigenverbrauch, wird es z. B. durch Extraktion oder Extraktionsdestillation abgetrennt. Im anderen Fall wird es bis zu Konzentrationen von 1 bis 0,1 Massen-% selektiv zu linearen Butenen hydriert. Zurück bleibt in beiden Fällen ein Kohlenwasserstoffgemisch (entsprechend Raffinat I bzw. hydriertes Crack-C₄), das neben den gesättigten Kohlenwasserstoffen (n-Butan und Isobutan) die Olefine (Isobuten, 1-Buten und 2-Butene) enthält. Dieses Kohlenwasserstoffgemisch wird mit Methanol in Gegenwart eines sauren Katalysators, meistens im Festbett angeordnet, umgesetzt, wobei gemäß der Gleichgewichtslage aus dem größten Teil des Isobutens MTBE entsteht. Die weitere Aufarbeitung dieses Reaktionsgemisches erfolgt durch Destillation in einer Destillationskolonne oder in einer Reaktivdestillationskolonne. Als Sumpfprodukt wird in beiden Fällen ein technisches MTBE (Kraftstoffqualität) erhalten,

Soll das so erhaltene technische MTBE höheren Reinheitsanforderungen genügen, so müssen die Verunreinigungen, hauptsächlich Methanol und Butanol abgetrennt werden.

In der Literatur sind mehrere Verfahren zur Abtrennung von Methanol aus MTBE-Strömen bekannt. Bei einigen Verfahren wird das Methanol vor oder während der destillativen Trennung des Reaktionsgemisches der MTBE-Synthese entfernt. Bei anderen Verfahren wird das Methanol direkt aus dem technischen MTBE abgetrennt.

US 3 726 942 beschreibt ein MTBE-Verfahren, bei dem aus dem nach der Synthese durch Destillation gewonnenen Roh-MTBE Methanol durch Wasserwäsche entfernt wird. US 3 846 088 offenbart ein Verfahren, bei dem zunächst Methanol durch Extraktion mit Wasser aus dem Roh-MTBE entfernt wird und anschließend das im MTBE gelöste Wasser durch Azeotropdestillation mit C₅-C₁₀-Paraffinen abgetrennt wird.

US 4 334 964 und US 4 544 776 beschreiben Verfahren, bei denen das Methanol aus dem Reaktionsgemisch der MTBE-Synthese vor der destillativen Auftrennung mit Wasser ausgewaschen wird.

US 4 605 787 offenbart ein MTBE-Verfahren, bei dem Methanol durch Adsorption an zeolithischen Molsieben (0,3 nm, 0,4 nm, 0,5 nm) aus dem durch Destillation gewonnenen Roh-MTBE abgetrennt wird.

Gemäß EP 0 317 918 wird Methanol vor oder während der destillativen MTBE-Abtrennung mit Hilfe einer Membran entfernt.

Nach DE 30 15 882 wird Methanol durch Extraktivdestillation aus Roh-MTBE abgetrennt. Als Extraktionsmittel wird dabei eine oder mehrere Verbindung aus den Gruppen der zwei-, dreiwertigen Alkohole, Aminoalkohole oder Dimethylformamid verwendet.

Die weitgehende Entfernung des im Roh-MTBE vorhandenen Methanols erfolgt nach US 4 256 465 durch Abdestillieren des MTBE/Methanol-Azeotrops, unter Erhalt eines hochreinen MTBE-Stroms. Das Azeotrop kann in den MTBE-Synthesereaktor zurückgefahren werden.

Die oben genannten Verfahren haben den Mangel, dass sie zwar Methanol und ggf. Wasser aus dem Roh-MTBE entfernen, nicht jedoch andere Begleitstoffe, wie beispielsweise C₈-Olefine, tert.- Butanol (TBA) oder 2-Methoxybutan (MSBE). Dies ist vermutlich darauf zurückzuführen, dass bei den bekannten Destillationsverfahren die Trennung so durchgeführt wird, dass MTBE als Sumpfprodukt (d. h. als Schwersieder) und ein Azeotrop von Methanol und MTBE bzw. ein Azeotrop von C₄-Kohlenwasserstoffen mit Methanol als Kopfprodukt erhalten werden. Diese Trennung ist zur Herstellung von sehr reinem MTBE nicht geeignet, da MTBE und die Oligomerisierungsprodukte von Butenen (Di-Isobuten) in unzulässigen Mengen im MTBE verbleiben.

Es bestand daher die Aufgabe, ein kostengünstiges Verfahren für die Herstellung von hochreinem Methyl-tert.-butylether aus technischen MTBE, (Kraftstoffqualität mit ca. 99 %iger Reinheit) zu entwickeln.

Es wurde nun gefunden, dass die gewünschte Reinheit von MTBE erhalten werden kann, indem technisches MTBE destillativ in drei Fraktionen getrennt wird, nämlich eine Leichtsiederfiaktion, eine Mittelfraktion und eine Hochsiederfraktion. Die Leichtsiederfraktion enthält MTBE, Methanol, Wasser sowie geringe Mengen an C₄- und C₅-Komponenten. Die Mittelfraktion besteht aus MTBE in der gewünschten Reinheit. Die Hochsiederfraktion enthält neben MTBE tert.-Butylalkohol (TBA), Olefine, die durch Oligomerisierung von Butenen entstanden sind, Derivate dieser Olefine und/oder sonstige Nebenprodukte.

Gegenstand der vorliegenden Erfindung ist daher ein Verfahren zur Herstellung von MTBE mit einer Reinheit größer als 99,7 % aus technischem MTBE durch fraktionierte Destillation, wobei das technische MTBE in eine Leichtsiederfraktion, enthaltend MTBE, Methanol und Wasser, eine Mittelfraktion enthaltend MTBE in einer Reinheit größer als 99,7 % und eine Hochsiederfraktion, enthaltend Butenoligomere und TBA getrennt wird. Hierbei wird die fraktionierte Destillation in einem Zweikolonnensystem, das aus einer Hauptkolonne und einer Seitenkolonne besteht, durchgeführt, wobei die Leichtsiederfraktion als Kopfprodukt und die Schwersiederfraktion als Sumpfprodukt der Hauptkolonne erhalten werden und die Mittelfraktion in der Seitenkolonne auf das MTBE mit einer Reinheit größer 99,7 % aufgereinigt wird.

Mit dem Verfahren der Erfindung kann MTBE auch mit einer Reinheit größer 99,8 % oder 99,9 % erzeugt werden. Die Prozentangaben beziehen sich immer auf Massen-%. Das verwendete technische MTBE wird üblicherweise als Kraftstoffzuschlag eingesetzt und hat eine Reinheit von maximal 99,0 %.

Die fraktionierte Destillation wird in mindestens zwei Kolonnen durchgeführt. Bevorzugt wird mindestens eine Trennwandkolonne eingesetzt.

Die Trennwandkolonne ist eine Kolonne, die durch eine vertikale Wand in zwei Teile getrennt wird. Hierdurch können zwei Destillationsschritte in einem Apparat durchgeführt werden, so dass eine Kolonne, sowie ein oder zwei Wärmetauscher eingespart werden können.

Das erfindungsgemäße Verfahren hat folgende Vorteile:

Das Investment und die Betriebskosten sind relativ gering. Die anfallenden Leicht- und Hochsiederfraktionen können ohne Verluste in eine Anlage zur Herstellung von technischen MTBE (Kraftstoffqualität) zurückgeführt werden.

Wenn das Produktionsmengenverhältnis von hochreinem MTBE zu MTBE mit Kraftstoffqualität gering ist, können sowohl die Leichtsieder-, als auch die Hochsiederfraktion in den Kraftstoff-MTBE-Strom zurückgeführt werden. Bei größeren Mengenverhältnissen kann die Leichtsiederfraktion in die MTBE-Synthese zurückgeführt werden, während die Hochsiederfraktion als Kraftstofflcomponente genutzt werden kann.

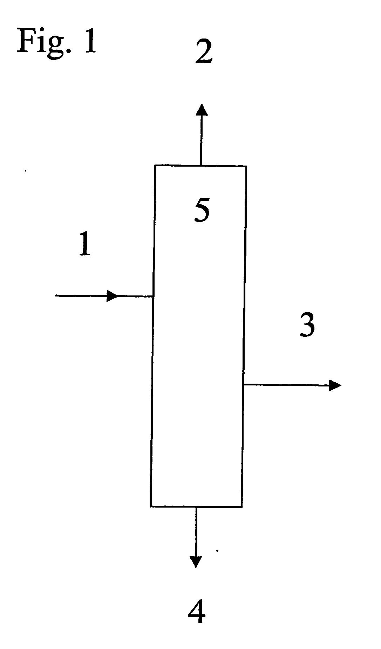

Erfindungsgemäß wird die Abtrennung des hochreinen MTBE in einer oder mehreren Destillationskolonnen durchgeführt. Einige möglichen Ausführungsarten sind schematisch in den Fig. 1-3 dargestellt.

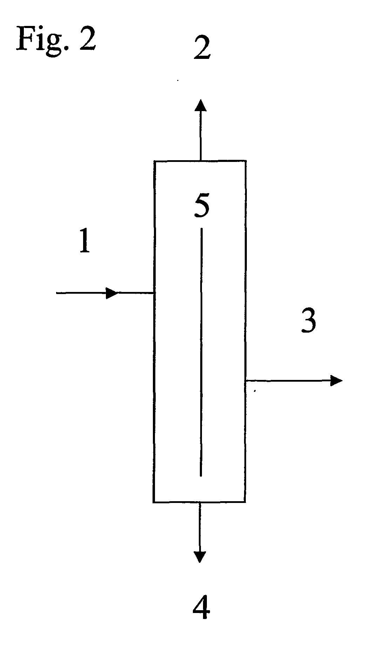

Die Fig. 1 und 2 verdeutlichen Verfahrensvarianten, bei denen die Gewinnung von hochreinem MTBE in einer Kolonne durchgeführt werden. Der Unterschied dieser beiden Varianten besteht darin, dass bei dem Verfahren nach Fig. 1 eine übliche Destillationskolonne und bei dem Verfahren nach Fig. 2 eine Trennwandkolonne verwendet wird. MTBE (technische Qualität) (1) wird in die Destillationskolonne (5) eingeleitet. Als Kopfprodukt (2) wird ein Gemisch aus MTBE, Methanol und Wasser abgezogen. Als Sumpfprodukt (4) fällt ein Gemisch aus MTBE, tert.-Butylalkohol (TBA), 2-Methoxybutan (MSBE) und höhere Olefine an. Das hochreine MTBE (3) wird als Seitenabzug entnommen.

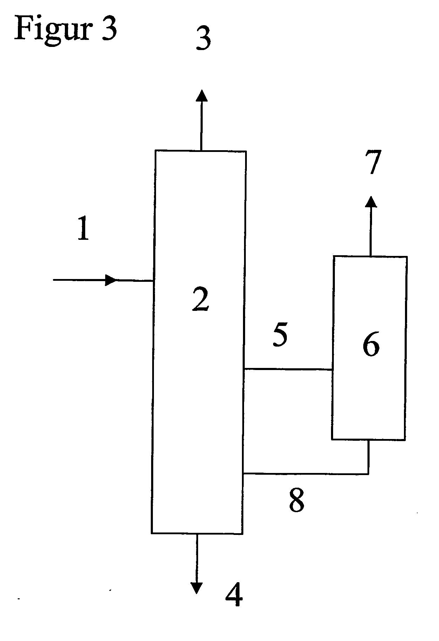

In der Verfahrensvariante nach Fig. 3 wird MTBE in Kraftstoffqualität (1) in die Destillationskolonne (2) eingeleitet. Methanol und Wasser werden zusammen mit einem Teil des MTBE als Kopfprodukt (3) und hochsiedende Begleitstoffe mit dem Sumpf (4) abgetrennt. Der Seitenabzug (5) wird in der Seitenkolonne (6) in hochreines MTBE (7) und in ein Sumpfprodukt (8), das in die erste Kolonne zurückgeführt wird, aufgetrennt. (Dabei kann der Seitenabzug (5) je nach Fahrweise der Nebenkolonne (6) unterhalb oder oberhalb des Zulaufes (1) flüssig oder dampfförmig abgezogen werden.)

Nach dem erfindungsgemäßen Verfahren wird Roh-MTBE in Kraftstoffqualität zu hochreinem MTBE aufgearbeitet. Als Einsatzprodukte eignen sich MTBE-Gemische, die als Begleitstoffe die Leichtsieder Methanol und Wasser, sowie die Hochsieder TBA, Oligomere von C₄-Olefinen, die davon abgeleiteten Alkohole und Methylether und 2-Methoxybutan (MSBE), enthalten können.

Vorzugsweise wird im erfindungsgemäßen Verfahren ein Roh-MTBE eingesetzt, das durch Umsetzung von Raffinat I oder von hydriertem Crack-C₄ mit Methanol nach bekannten Verfahren gewonnen wird. Dieses MTBE (Kraftstoffqualität) hat typischerweise einen MTBE-Gehalt von 98 bis 99,0 Massen-%, einen Gehalt an C₈-Kohenwasserstoffe von weniger als 0,5 Massen-%, einen Gehalt an TBA von weniger als 1 Massen-%, einen Gehalt an Methanol von weniger als 1-Massen-% und einen Wassergehalt von weniger als 0,05 Massen-% sowie einen Gehalt von 2-Methoxybutan (MSBE) von bis zu 0,5 Massen-%.

Mit dem erfindungsgemäßen Verfahren kann ein Rein-MTBE (Reinheit größer 99,7 %) mit einem Gehalt an 2-Methoxybutan mit weniger als 500 Massen-ppm, insbesondere 100 bis 300 Massen-ppm, erzeugt werden. Hierzu ist es zweckmäßig, aber nicht unbedingt erforderlich, eine MTBE-Qualität mit weniger als 0,3 Massen-% 2-Methoxybutan als Einsatzstoff zu verwenden. Die Herstellung eines MTBE (Kraftstoffqualität) mit einem 2-Methoxybutangehalt (MSBE) von unter 0,25 Massen-% wird beispielsweise in DE 101 02 082.1 beschrieben.

Optional können auch Roh-MTBE-Gemische, die auf andere Art hergestellt wurden, wie beispielweise aus TBA und Methanol, verwendet werden.

Es sei darauf hingewiesen, dass nach dem erfindungsgemäßen Verfahren auch Roh-MTBE-Gemische zu hochreinem MTBE aufgearbeitet werden können, deren Zusammensetzung in dieser oder jener Beziehung nicht den obigen Angaben entspricht.

Die destillative Gewinnung von hochreinem MTBE wird in einer oder mehreren Kolonnen mit Einbauten, die aus Böden, rotierenden Einbauten, ungeordneten und/oder geordneten Packungen bestehen, durchgeführt.

Bei den Kolonnenböden kommen folgende Typen zum Einsatz:
- Böden mit Bohrungen oder Schlitzen in der Bodenplatte.
- Böden mit Hälsen oder Kaminen, die von Glocken, Kappen oder Hauben überdeckt sind.
- Böden mit Bohrungen in der Bodenplatte, die von beweglichen Ventilen überdeckt sind.
- Böden mit Sonderkonstruktionen.

In Kolonnen mit rotierenden Einbauten wird der Rücklauf entweder durch rotierende Trichter versprüht oder mit Hilfe eines Rotors als Film auf einer beheizten Rohrwand ausgebreitet.

In dem erfindungsgemäßen Verfahren verwendete Kolonnen können regellose Schüttungen mit verschiedenen Füllkörpern aufweisen. Sie können aus fast allen Werkstoffen - Stahl, Edelstahl, Kupfer, Kohlenstoff Steingut, Porzellan, Glas, Kunststoffen usw. - und in verschiedenen Formen - Kugeln, Ringen mit glatten oder profilierten Oberflächen, Ringen mit Innenstegen oder Wanddurchbrüchen, Drahtnetzringen, Sattelkörper und Spiralen - bestehen.

Packungen mit regelmäßiger Geometrie können z.B. aus Blechen oder Geweben bestehen. Beispiele solcher Packungen sind Sulzer Gewebepackungen BX aus Metall oder Kunststoff, Sulzer Lamellenpackungen Mellapack aus Metallblech, Strukturpackungen von Sulzer (Optiflow), Montz (BSH) und Kühni (Rombopack).

Das erfindungsgemäße Verfahren kann bei Unterdruck, Normaldruck oder Überdruck durchgeführt werden. Ein bevorzugter Druckbereich ist 1,5-10 bar, insbesondere 2-6 bar.

Die Leichtsiederfraktion, die im Wesentlichen aus einem MTBE/Methanol-Azeotrop besteht, wird bei einer Kopftemperatur abgezogen, die zwischen dem Siedepunkt des MTBE/Methanol-Azeotrops und dem Siedepunkt des MTBE bei Destillationsdruck liegt.

Die Schwersiederfraktion, die hauptsächlich aus MTBE und TBA besteht, wird bei einer Temperatur abgezogen, die zwischen dem Siedepunkt des MTBE und dem Siedepunkt des TBA bei Destillationsdruck liegt.

In einer speziellen Ausführung der Erfindung wird die fraktionierte Destillation in einem Zweikolonnensystem, das aus einer Hauptkolonne und einer Seitenkolonne besteht, durchgeführt, wobei die Leichtsiederfraktion als Kopfprodukt und die Schwersiederfraktion als Sumpfprodukt der Hauptkolonne erhalten werden und die Mittelfraktion in der Seitenkolonne auf das gewünschte MTBE mit einer Reinheit von 99,7 % aufgereinigt wird.

Das MTBE mit einer Reinheit größer 99,7 % kann, je nach Fahrweise als Kopf oder Sumpfprodukt der Seitenkolonne erhalten werden. Die jeweils anderen Fraktionen, die nicht das gewünschte MTBE enthalten, können optional in die Hauptkolonne zurückgeführt werden. Eine dieser Varianten ist in Fig. 3 skizziert.

Bei einer Anordnung des Seitenabzuges oberhalb des Feedbodens hat die Hauptkolonne bevorzugt 20 bis 100 theoretische Trennstufen, insbesondere 35 bis 70 Trennstufen. Davon entfallen in der Regel 5 bis 30 theoretische Trennstufen, insbesondere 10 bis 20 Trennstufen auf den Verstärkungsteil, 5 bis 20 theoretische Trennstufen, insbesondere 5 bis 15 Trennstufen auf den Mitteilteil, sowie 10 bis 50 theoretische Trennstufen, insbesondere 20 bis 35 Trennstufen auf den Abtriebsteil.

Die Hauptkolonne wird bevorzugt mit einem Rücklaufverhältnis von 5 bis 80, insbesondere mit einem von 10 bis 50 kg/kg betrieben.

Die Seitenkolonne hat meist 10 bis 50 theoretische Trennstufen, insbesondere 20 bis 35 Trennstufen. Diese Kolonne wird bevorzugt mit einem Rücklaufverhältnis von 1 bis 20, insbesondere mit einem von 2 bis 10 kg/kg betrieben.

Wird der Seitenabzug der Hauptkolonne unterhalb des Feedbodens angeordnet, hat die Hauptkolonne bevorzugt 25 bis 130 theoretische Trennstufen, insbesondere 50 bis 90 Trennstufen. Davon entfallen in der Regel 5 bis 30 theoretische Trennstufen, insbesondere 10 bis 20 Trennstufen auf den Verstärkungsteil, 10 bis 50 theoretische Trennstufen, insbesondere 20 bis 35 Trennstufen auf den Mittelteil, sowie 10 bis 50 theoretische Trennstufen, insbesondere 20 bis 35 Trennstufen auf den Abtriebsteil.

Bei dieser Anordnung wird die Hauptkolonne bevorzugt mit einem Rücklaufverhältnis von 30 bis 600, insbesondere mit einem von 60 bis 300 kg/kg betrieben

Die Seitenkolonne hat dabei meist 5 bis 20 theoretische Trennstufen, insbesondere 5 bis 15 Trennstufen. Diese Kolonne wird bevorzugt mit einem Aufdampfverhältnis von 0,2 bis 2,5, insbesondere mit einem von 0,4 bis 1,5 kg/kg betrieben.

Die abgetrennte Leichtsiedenraktion enthält bis zu 75 % MTBE. Der Rest ist im Wesentlichen Methanol. Darüber hinaus können in ihr geringe Mengen an Wasser sowie C₄- und C₅-Kohlenwasserstoffe enthalten sein. Dieser Strom kann in den Synthesereaktor einer MTBE-Anlage eingespeist werden. Wenn nur ein kleiner Teil des Roh-MTBE (Kraftstoff qualität) aus einer MTBE-Anlage zu hochreinem MTBE aufgearbeitet wird, kann die Leichtsiederfraktion als Kraftstoffkomponente verwendet werden.

Es ist möglich, die Leicht- und/oder die Schwersiederfraktion in die fraktionierte Destillation, optional unter Ausschleusung eines Teilstroms zurückzuführen.

Die Hochsiederfraktion besteht zu über 70 %, meistens zu über 85 % aus MTBE. Weiterhin enthält sie TBA, 2-Methoxybutan, Oligomere von Butenen und deren Derivate. Diese Fraktion kann, gegebenenfalls nach Hydrierung der darin enthaltenen Olefine, ebenfalls als Kraftstoffkomponente verwendet werden.

Das nach dem erfindungsgemäßen Verfahren gewonnene hochreine MTBE hat eine Reinheit von über 99,7 %, bevorzugt 99,8 %, besonders bevorzugt 99,9 %. Der Gehalt an Kohlenwasserstoffen liegt unter 0,1, bevorzugt unter 0,02 Massen-%, der Gehalt an TBA unter 0,05, bevorzugt unter 0,025 Massen-%, der Gehalt an Methanol unter 0,02, bevorzugt unter 0,01 Massen-% und der Gehalt an Wasser unter 200, bevorzugt unter 100 Massen-ppm. Wegen seiner hohen Reinheit kann dieses MTBE im Pharmabereich, in der Analytik, als Löse- und Extraktionsmittel eingesetzt werden. Besonders geeignet ist es als Lösemittel in organischen Synthesen, beispielsweise bei der Herstellung von metallorganischen Verbindungen und deren Umsetzungen. MTBE, das darüber hinaus einen Gehalt an 2-Methoxybutan von weniger als 500 Massen-ppm aufweist, ist besonders gut für die Herstellung von hochreinem Isobuten (durch Rückspaltung) in Isobuten und Methanol geeignet.

Ein solches Verfahren schließt sich zweckmäßig dem erfindungsgemäßen Verfahren zur MTBE-Aufreinigung an. Ein Verfahren zur Herstellung von hochreinem Isobuten durch katalytische Spaltung nach einem der Ansprüche 1 bis 10 hergestellten MTBE mit einer Reinheit von größer 99,7 % ist daher auch Gegenstand der vorliegenden Erfindung.

Die folgenden Beispiele sollen die Erfindung erläutern, ohne ihre Anwendungsbreite einzuschränken, die sich aus der Beschreibung und den Patentansprüchen ergibt.

### Beispiel 1: Abtrennung der Leichtsieder aus Roh-MTBE (Kraftstoffqualität)

Die destillative Abtrennung der Leichtsieder wurde kontinuierlich in einer ersten Technikumskolonne mit einem Durchmesser von 50 mm durchgeführt. Die Kolonne war mit der Gewebepackung Sulzer BX gefüllt und hatte 25 theoretische Trennstufen.

Die Betriebsparameter waren wie folgt:

| | |
|---|---|
| Zulaufmenge | 14 kg/h |
| Abgezogene Destillatmenge | 0,5 kg/h |
| Abgezogene Sumpfmenge | 13,5 kg/h |
| Zulauftemperatur | 80°C |
| Kopftemperatur | 80,8 °C |
| Sumpftemperatur | 93,5 °C |
| Druck (Kopf) | 3 bar |
| Zulaufboden | 8. theoretische Trennstufe von unten |
| Dampfbelastungsfaktor | 0,75 Pa^{0,5} |
| Flüssigkeitsbelastung | 10-20 m³/(m²*h) |
| Rücklaufverhältnis | 6 kg/kg |

Die Zusammensetzung von Kolonnenzufluss, Destillat und Sumpfprodukt wurde in Tabelle 1 zusammengefasst.

**Tabelle 1: Zusammensetzung der Stoffströme in Kolonne 1**

| **Stoff** | **Zusammensetzung in Massen-%** | | |
|---|---|---|---|
| | **Kolonnenzulauf** | **Sumpfprodukt** | **Destillat** |
| C₄/C₅-Kohlenwasserstoffe | 0,299 | 0,02 | 8,477 |
| MTBE | 97,897 | 98,741 | 73,164 |
| 2-Methoxybutan | 0,299 | 0,309 | 0,017 |
| Methanol | 0,598 | 0,003 | 18,039 |
| tert.-Butanol | 0,798 | 0,825 | 0,001 |
| Wasser | 0,010 | < 0,001 | 0,302 |
| C₈-Kohlenwasserstoffe | 0,100 | 0,103 | < 0,001 |

Es wurde ein praktisch Wasser- und Methanol-freies MTBE als Sumpfprodukt abgetrennt.

### Beispiel 2: Abtrennung des hochreinen MTBE

Die destillative Gewinnung des hochreinen MTBE aus dem Sumpfprodukt der ersten Kolonne wurde kontinuierlich in einer zweitenTechnikumskolonne mit einem Durchmesser von 50 mm durchgeführt. Die Kolonne war mit der Gewebepackung Sulzer BX gefüllt und hatte 50 theoretische Trennstufen.

Die Betriebsparameter waren wie folgt:

| | |
|---|---|
| Zulaufmenge | 2,5 kg/h |
| Abgezogene Destillatmenge | 2,1 kg/h |
| Abgezogene Sumpfmenge | 0,4 kg/h |
| Zulauftemperatur | 94,7 °C |
| Kopftemperatur | 71,7 °C |
| Sumpftemperatur | 74,0 °C |
| Druck (Kopf) | 1,7 bar |
| Zulaufboden | 24. Trennstufe von unten |
| Dampfbelastungsfaktor | 0,75 Pa^{0,5} |
| Flüssigkeitsbelastung | 10m³/(m²/h) |
| Rücklaufverhältnis | 6 kg/kg |

Die Zusammensetzung von Kolonnenzufluss, Destillat und Sumpfprodukt wurde in Tabelle 2 zusammengefasst.

**Tabelle 2: Zusammensetzung der Stoffströme in Kolonne 2**

| **Stoff** | **Zusammensetzung in Massen-%** | | |
|---|---|---|---|
| | **Kolonnenzulauf** | **Sumpfprodukt** | **Destillat** |
| C₄/C₅-Kohlenwasserstoffe | 0,021 | <0,001 | 0,025 |
| MTBE | 98,739 | 92,040 | 99,922 |
| 2-Methoxybutan | 0,309 | 1,775 | 0,050 |
| Methanol | 0,003 | <0,001 | 0,004 |
| tert.-Butanol | 0,825 | 5,500 | <0,001 |
| Wasser | <0,001 | <0,001 | <0,001 |
| C₈-Kohlenwasserstoffe | 0,103 | 0,687 | <0,001 |

Es wurde ein MTBE in einer Reinheit von über 99,9 % als Destillat erhalten.Wegen seines geringen Gehalt an protischen Begleitstoffen (Wasser, Methanol, tert.-Butanol) ist dieses MTBE insbesondere ein gutes Lösemittel für metallorganische Synthesen. Wegen des geringen Gehalts an 2-Methoxybutan ist dieses MTBE besonders für die Herstellung von hochreinem Isobuten geeignet.

## Patentansprüche

1. Verfahren zur Herstellung von MTBE mit einer Reinheit größer als 99,7 % aus technischem MTBE durch fraktionierte Destillation,
**dadurch gekennzeichnet,**
**dass** das technische MTBE in eine Leichtsiederfraktion, enthaltend MTBE, Methanol und Wasser, eine Mittelfraktion enthaltend MTBE in einer Reinheit größer als 99,7 % und eine Hochsiederfraktion, enthaltend TBA und Butenoligomere, getrennt wird, wobei die fraktionierte Destillation in einem Zweikolonnensystem, das aus einer Hauptkolonne und einer Seitenkolonne besteht, durchgeführt wird, und wobei die Leichtsiederfraktion als Kopfprodukt und die Schwersiederfraktion als Sumpfprodukt der Hauptkolonne erhalten werden und die Mittelfraktion in der Seitenkolonne auf das MTBE mit einer Reinheit größer 99,7 % aufgereinigt wird

2. Verfahren nach Anspruch 1,
**dadurch gekennzeichnet,**
**dass** die fraktionierte Destillation in mindestens einer Trennwandkolonne durchgeführt wird.

3. Verfahren nach Anspruch 1 oder 2,
**dadurch gekennzeichnet,**
**dass** das MTBE mit einer Reinheit größer 99,7 % als Kopfprodukt der Seitenkolonne erhalten wird.

4. Verfahren nach Anspruch 3,
**dadurch gekennzeichnet,**
**dass** das MTBE mit einer Reinheit größer 99,7 % als Sumpfprodukt der Seitenkolonne erhalten wird.

5. Verfahren nach einem der Ansprüche 1 bis 4,
**dadurch gekennzeichnet,**
**dass** die Fraktion der Seitenkolonne, die nicht das MTBE mit einer Reinheit größer 99,7 % enthält, in die Hauptkolonne zurückgeführt wird.

6. Verfahren nach einem der Ansprüche 1 bis 5,
**dadurch gekennzeichnet,**
**dass** das MTBE mit einer Reinheit größer 99,7 % einen Gehalt an 2-Methoxybutan von weniger als 500 Massen-ppm aufweist.

7. Verfahren nach einem der Ansprüche 1 bis 6,
**dadurch gekennzeichnet,**
**dass** die Leichtsiederfraktion in die fraktionierte Destillation zurückgeführt wird.

8. Verfahren nach einem der Ansprüche 1 bis 7,
**dadurch gekennzeichnet,**
**dass** die Hochsiederfraktion in die fraktionierte Destillation zurückgeführt wird.

9. Verfahren zur Herstellung von hochreinem Isobuten durch katalytische Spaltung des nach einem der Ansprüche 1 bis 8 hergestellten MTBE mit einer Reinheit von größer 99,7 %.

## Claims

1. Process for preparing MTBE having a purity of greater than 99.7% from technical grade MTBE by fractional distillation,
**characterized in that**
the technical grade MTBE is separated into a low boiler fraction comprising MTBE, methanol and water, a middle fraction comprising MTBE in a purity of greater than 99.7% and a high boiler fraction comprising TBA and butene oligomers, the fractional distillation being carried out in a two-column system which consists of a main column and a side column, and the low boiler fraction being obtained as the top product and the high boiler fraction as the bottom product of the main column and the middle fraction being purified in the side column to give the MTBE having a purity of greater than 99.7%.

2. Process according to claim 1,
**characterized in that**
the fractional distillation is carried out in at least one dividing wall column.

3. Process according to claim 1 or 2,
**characterized in that**
the MTBE having a purity of greater than 99.7% is obtained as the top product of the side column.

4. Process according to claim 3,
**characterized in that**
the MTBE having a purity of greater than 99.7% is obtained as the bottom product of the side column.

5. Process according to any of claims 1 to 4,
**characterized in that**
the fraction of the side column which does not contain the MTBE having a purity of greater than 99.7% is recycled into the main column.

6. Process according to any of claims 1 to 5,
**characterized in that**
the MTBE having a purity of greater than 99.7% has a 2-methoxybutane content of less than 500 ppm by mass.

7. Process according to any of claims 1 to 6,
**characterized in that**
the low boiler fraction is recycled into the fractional distillation.

8. Process according to any of claims 1 to 7,
**characterized in that**
the high boiler fraction is recycled into the fractional distillation.

9. Process for preparing highly pure isobutene by catalytically cleaving the MTBE having a purity of greater than 99.7% prepared according to any of claims 1 to 8.

## Revendications

1. Procédé de préparation de MTBE présentant une pureté supérieure à 99,7% à partir de MTBE technique par distillation fractionnée, **caractérisé en ce que** le MTBE technique est séparé en une fraction à bas point d'ébullition, contenant du MTBE, du méthanol et de l'eau, une fraction moyenne contenant du MTBE en une pureté supérieure à 99,7% et une fraction à point d'ébullition élevé, contenant du TBA et des oligomères de butène, la distillation fractionnée étant réalisée dans un système à deux colonnes qui est constitué par une colonne principale et une colonne latérale, et la fraction à bas point d'ébullition étant obtenue comme produit de tête et la fraction à point d'ébullition élevé étant obtenue comme produit de fond de la colonne principale et la fraction moyenne étant purifiée dans la colonne latérale en MTBE en une pureté supérieure à 99,7%.

2. Procédé selon la revendication 1, **caractérisé en ce que** la distillation fractionnée est réalisée dans au moins une colonne à paroi de séparation.

3. Procédé selon la revendication 1 ou 2, **caractérisé en ce qu'**on obtient du MTBE présentant une pureté supérieure à 99,7% comme produit de tête de la colonne latérale.

4. Procédé selon la revendication 3, **caractérisé en ce qu'**on obtient du MTBE présentant une pureté supérieure à 99,7% comme produit de fond de la colonne latérale.

5. Procédé selon l'une quelconque des revendications 1 à 4, **caractérisé en ce que** la fraction de la colonne latérale qui ne contient pas le MTBE en une pureté supérieure à 99,7%, est recyclée dans la colonne principale.

6. Procédé selon l'une quelconque des revendications 1 à 5, **caractérisé en ce que** le MTBE présentant une pureté supérieure à 99,7% présente une teneur en 2-méthoxybutane inférieure à 500 ppm en masse.

7. Procédé selon l'une quelconque des revendications 1 à 6, **caractérisé en ce que** la fraction à bas point d'ébullition est recyclée dans la distillation fractionnée.

8. Procédé selon l'une quelconque des revendications 1 à 7, **caractérisé en ce que** la fraction à point d'ébullition élevé est recyclée dans la distillation fractionnée.

9. Procédé pour la préparation d'isobutène de haute pureté par dissociation catalytique du MTBE présentant une pureté supérieure à 99,7% préparé selon l'une quelconque des revendications 1 à 8.
